Europäisches Patentamt

(19) European Patent Office

Office européen des brevets

(11) Publication number : **0 372 888 B1**

(12) # EUROPEAN PATENT SPECIFICATION

(45) Date of publication of patent specification :
**22.07.92 Bulletin 92/30**

(51) Int. Cl.⁵ : **A61K 31/71, A61K 47/08**

(21) Application number : **89312625.0**

(22) Date of filing : **04.12.89**

(54) **Doxorubicin aqueous solutions.**

(30) Priority : **05.12.88 US 279627**

(43) Date of publication of application :
**13.06.90 Bulletin 90/24**

(45) Publication of the grant of the patent :
**22.07.92 Bulletin 92/30**

(84) Designated Contracting States :
**AT BE CH DE ES FR GB GR IT LI LU NL SE**

(56) References cited :
**EP-A- 0 299 527**
**GB-A- 2 178 311**

(73) Proprietor : **Bristol-Myers Squibb Company**
**345 Park Avenue**
**New York, N.Y. 10154 (US)**

(72) Inventor : **Kaplan, Murray A.**
**1026 Glencove Road**
**Syracuse New York 13206 (US)**
Inventor : **Perrone, Robert K.**
**7353 Tomwood Drive**
**Liverpool New York 13090 (US)**
Inventor : **Bogardus, Joseph B.**
**8239 Penstock Way**
**Manlius New York 13104 (US)**

(74) Representative : **Jones, Alan John et al**
**CARPMAELS & RANSFORD 43 Bloomsbury**
**Square**
**London, WC1A 2RA (GB)**

EP 0 372 888 B1

## Description

The present invention relates to stable, ready-to-use aqueous solutions of doxorubicin suitable for parenteral administration.

Doxorubicin is an antineoplastic antibiotic which is isolated from the fermentation of Streptomyces peucetius var. caesius. It can also be synthesized chemically from daunorubicin.

The commercial lyophilized dosage form of doxorubicin (marketed by Adria Laboratories as Adriamycin®) is supplied as the hydrochloride salt in 10, 20 and 50 mg vials in combination with lactose at 50, 100 and 250 mg, respectively. Vials are reconstituted with Sodium Chloride Injection, USP (0.9%) or Sterile Water for Injection, USP, to give a final concentration of 2 mg/ml of doxorubicin HCl and a pH range of about 4-6. The reconstituted lyophilized solution is reported by the manufacturer to be stable ($\leq$ 10% potency loss) for only 24 hours at room temperature and 48 hours under refrigeration.

It would be desirable to have a solution dosage form of doxorubicin which would not require reconstitution prior to use. Improper reconstitution of a lyophilized product sometimes results in the formation of air-borne droplets ("blow-back") which, in the case of a potent antitumor agent such as doxorubicin, may be a health hazard to the personnel making up the solution for injection. Also production of the present lyophilized doxorubicin HCl dosage form is quite costly, and a solution dosage form would be expected to have a lower cost of goods.

Doxorubicin HCl has recently been made commercially available as a preservative-free aqueous solution. Despite the convenience of a ready-to-use solution, the present aqueous solution is still not as stable as one would desire. The manufacturer indicates that the ready-to-use solution is stable for 18 months at 4°C. At the end of this time period, the solution may contain up to 10% of undesirable and potentially toxic degradation products.

A study carried out by Hoffman, et al. and reported in Am. J. Hosp. Pharm. 36: 1536-1538, 1979 indicates that reconstituted solutions of doxorubicin HCl are stable ($\leq$ 10% potency loss) for up to six months when refrigerated at 4°C. Ketcyhum, et al. in Am. J. Intrav. Ther. Clin. Nutri. 8: 15-18, 1981, state that a reconstituted solution of doxorubicin HCl in 0.9% NaCl solution remains stable at room temperature or 5°C for seven days. Despite such indications that reconstituted doxorubicin HCl may be sufficiently stable for up to six months at 4°C, this is still insufficient for a commercial solution dosage form.

Janssen, et al. in Int. J. Pharmaceutics 23: 1-11, 1985, report that doxorubicin HCl appears to have its maximum stability at pH 4 (Tris or phosphate buffers using HCl to adjust pH). Poochikian, et al. in Am. J. Hosp. Pharm. 38: 483-486, 1981, note that the stability of doxorubicin HCl is pH dependent and that the optimum stability is achieved when the reconstitution solvent is 5% Dextrose Injection, USP, having a pH of approximately 4.5.

U.K. Patent Application 2,178,311 A discloses solutions of doxorubicin HCl in aqueous and non-aqueous solvent systems. There is no disclosure, however, of the use of the antioxidant, sodium acetone bisulfite, to stabilize such solutions.

European Published Application 273,603 A2 discloses solutions of doxorubicin HCl in aqueous and non-aqueous solvent systems and appears to be substantially equivalent to U.K. 2,178,311 A, except for limiting the pH range to 2.5 to 4.0 instead of the broader pH range of 2.5 to 6.5 disclosed in UK 2,178,311.

U.S. Patent 4,675,311 discloses aqueous solutions of doxorubicin HCl containing a co-solubilizing agent selected from the group comprising a hydroxy-, mercapto-, or amino-substituted benzoic acid, an alkali metal salt thereof, a $C_1$-$C_4$ alkyl ester thereof, a ring-halogenated methyl-substituted phenol, an amino acid, and mixtures thereof.

Applicants have previously filed co-pending EP-A-88111433.4 which discloses and claims stable, ready-to-use aqueous solutions of doxorubicin HCl containing an antioxidant selected from $\alpha$-tocopherol, sodium formaldehyde bisulfite and tert-butyl hydroquinone.

The antioxidant, sodium acetone bisulfite, used in the present invention has been previously utilized as a stabilizing agent for other pharmaceutical products. For example, Canadian Patent 981,182 discloses use of sodium acetone bisulfite in combination with thioglycerol and ascorbic acid to stabilize $\gamma$-epinephrine aqueous solutions. Sodium acetone bisulfite is also used as a preservative in other pharmaceutical solutions, e.g. pentazocine lactate and procaine hydrochloride.

It is an object of the present invention to provide a ready-to-use aqueous solution of a physiologically acceptable salt of doxorubicin which is stable ($\leq$ 10% potency loss) for more than three years at 4°C.

The present invention provides a stable, sterile, ready-to-use solution in a sealed container suitable for parenteral administration containing water, a physiologically acceptable salt of doxorubicin, a stabilizing amount of sodium acetone bisulfite of about 0.1 to 10 mg/ml, and, optionally, an amount of a physiologically acceptable acid or base necessary to adjust the solution pH to within the range of about 2.5 to 6.0.

This invention relates to stable, aqueous, ready-to-use solutions of a physiologically acceptable salt of doxorubicin suitable for parenteral administration. Although concentrations of from 0.1 to 20 mg/ml of the doxorubicin salt may be employed in the solutions, it is preferred to use from 0.2 to 20 mg/ml, more preferably 2 to 5 mg/ml and, most preferably, 2 mg/ml.

Although the present invention is generally described below in terms of doxorubicin hydrochloride, any physiologically acceptable salt of doxorubicin may be used for preparing the aqueous solutions. Examples of suitable salts include, for example, the salts with mineral inorganic acids such as hydrochloric, hydrobromic, sulfuric, phosphoric, and nitric, and the salts with organic acids such as acetic, succinic, tartaric, ascorbic, citric, glutamic, benzoic, methanesulfonic and 2-hydroxyethanesulfonic,. The preferred physiologically acceptable salt is doxorubicin hydrochloride.

The pH of the solutions should be in the range of about 2.5 to 6.0. If pH adjustment is necessary to achieve this range, common physiologically acceptable acids and bases can be added. For example, the solution pH can be lowered by addition of suitable organic or inorganic acids such as hydrochloric, hydrobromic, sulfuric, phosphoric, nitric, acetic, succinic, tartaric, ascorbic, citric, glutamic, benzoic, methanesulfonic and 2-hydroxyethanesulfonic. Solution pH may be raised by addition of physiologically acceptable bases such as sodium carbonate, sodium bicarbonate or sodium hydroxide. A particularly preferred acid is citric acid since this reagent acts as a chelation agent as well as providing pH control. Without pH adjustment the solutions will generally be in the pH range of about 4.5 to 6.0. One preferred embodiment comprises solutions having a pH of from about 3.0 to 3.5. Another preferred embodiment comprises solutions having a pH in the range of about 4.5 to 6.0. This latter pH provides a solution which may have less irritation potential than the more acidic ready-to-use aqueous doxorubicin HCl solution commercially available.

It is also necessary for optimum stability of doxorubicin aqueous solutions, especially in the pH range of about 4 to 6 and at elevated temperatures which may be encountered at some time during the life of the product, e.g. during shipping, to utilize an antioxidant. We have tested a number of antioxidants including $\alpha$-tocopherol, tert-butylhydroquinone (TBHQ), sodium formaldehyde bisulfite, butylated hydroxy anisole (BHA), butylated hydroxy toluene (BHT), sodium bisulfite and propyl gallate. Of the antioxidants tested, only $\alpha$-tocopherol, tert-butylhydroquinone and sodium formaldehyde bisulfite resulted in the desired stability at elevated temperatures as well as at 4 °C. This finding was disclosed and claimed in our co-pending application EP-A-88111433.4.

Later work unexpectedly found that another antioxidant, sodium acetone bisulfite, gave even superior stabilizing properties to doxorubicin solutions, especially at pH 4.5 to 6.0 at which the other antioxidants are not effective, and it is this discovery which is the subject of the present invention. Generally, an amount of sodium acetone bisulfite is employed in the range of about 0.1 to 10 mg/ml. The preferred concentration of antioxidant is about 0.1 to 2 mg/ml with about 1 mg/ml being most preferred for solutions containing 2 mg/ml doxorubicin salt.

As noted above sodium acetone bisulfite is unique among the antioxidants in that it stabilizes doxorubicin solutions over a wide pH range, i.e. about 2.5 to 6.0, whereas the antioxidants disclosed and claimed in our co-pending application work only in the pH range of about 3 to 4. Elevated temperature data indicates that at 40°C essentially no potency loss is seen after four weeks with pH 5.1 solutions of the present invention, the approximate pH of the commercial doxorubicin HCl reconstituted lyophile.

In our studies the use of buffers at pH 4.0 such as acetate and citrate buffers had a deleterious effect on stability of the solutions and the presence of surfactants such as Tween-80 did not improve stability.

European Patent Application 273,603 A2 discloses the use of sugars and sugar alcohols as tonicity adjustment agents. While such agents have not been found to increase the stability of doxorubicin solutions, they may be used, if desired, in the solutions of the present invention. Suitable tonicity adjustment agents may be, for example, physiologically acceptable inorganic chlorides, e.g. sodium chloride (preferably 0.9% by weight sodium chloride), dextrose, lactose, mannitol or sorbitol. When a sugar or sugar alcohol such as dextrose, lactose, mannitol or sorbitol is used as a tonicity adjustment agent, preferably from 2.5 to 7.5% by weight is used, more preferably from 4 to 6% by weight and most preferably 5% by weight. The tonicity adjustment agent is preferably employed in an amount which will provide a solution with osmolarity of 200 to 400 mOsm/liter of solution.

Despite reports of potency losses of doxorubicin HCl solutions via photolytic degradation and absorption to container walls, we have not noted any significant loss of potency due to container absorption or sensitivity to normal room light with our solutions. Glass vials may be used as the container or, alternatively, the solution may be filled into hypodermic syringes of pre-determined volume to provide a ready-to-use solution which is also ready to inject or mix with intravenous fluids.

We have also found that deaeration of the solvent water and inert gas purging, for example with nitrogen, argon or helium, of the filled vials before they are sealed contributes to optimum stability and is most preferred. In contrast to the antioxidants previously tested, however, including $\alpha$-tocopherol, tert-butylhydroquinone and sodium formaldehyde bisulfite, inert gas purging was found to be less critical when sodium acetone bisulfite is employed. This is another indication of the unusual effectiveness of this particular antioxidant for stabilization of doxorubicin solutions. Because of this property, manufacturing control will be simplified since air need not be totally eliminated.

As an example of a stable, ready-to-use aqueous solution of doxorubicin HCl, 2.0 mg/ml doxorubicin HCl, 0.2 mg/ml citric acid (anhydrous) 1.0 mg/ml of sodium acetone bisulfite and sterile water are added to a Type I flint vial, the vial is purged with nitrogen and then sealed with a suitable Teflon coated stopper. Vials containing larger concentrations of doxorubicin HCl are prepared in a similar manner.

Elevated temperature studies using high performance liquid chromatographic (HPLC) assay indicate that our ready-to-use aqueous solutions have a shelf-life ($\leq$ 10% potency loss) of greater than three years at 4 °C. Based on our elevated temperature studies, the commercially available preservative-free doxorubicin HCl aqueous solution would have a maximum shelf-life of about 1.5 to 2.5 years at 4°C. Thus, the solutions provided by the present invention have a greater shelf-life than the heretofore available ready-to-use doxorubicin solutions. They also show far less doxorubicin degradation products (glycone and aglycone) than prior art products when aged over a wide temperature range.

The ready-to-use solutions of the present invention are employed for the treatment of cancer in the same manner as the commercially available doxorubicin HCl aqueous solution or reconstituted lyophilized products.

The following example illustrates but does not limit in any way the present invention.

Example 1

Formula

| Ingredient | mg/ml |
|---|---|
| Doxorubicin HCl | 2.0 |
| Anhydrous citric acid | 0.2 |
| Sodium acetone bisulfite | 1.0 |
| Deaerated water for injection, U.S.P. (or equivalent) | q.s. to 1 ml |

Nitrogen (aqueous and headspace saturated with nitrogen)

Procedure:

1. Using a suitable glass container, deaerate U.S.P. Water for Injection, or its equivalent, by boiling or sonication under vacuum.
2. Saturate the cooled water (20°-25°C) and container head-space with nitrogen. Store protected from air.
3. Place approximately 70-80% of the required volume of the nitrogen-saturated water in a suitable volume-tared glass container. For this and the following, maintain a heavy nitrogen purge, deep within the liquid and with slow vortex-free stirring.
4. Slowly add and dissolve the required amount of sodium acetone bisulfite.
5. Cautiously and slowly add and dissolve the required amount of the doxorubicin HCl.
6. Prepare a 10 mg/ml solution of anhydrous citric acid in deaerated, nitrogen-saturated water.
Slowly add the citric acid solution until a pH of 3-3.5 is obtained. Note the amount of solution used. Assure that the pH is stabilized at 3-3.5.
7. Stop and remove the stirrer. Stop the nitrogen flow.
8. Add deaerated, nitrogen-saturated water, q.s. to the final required volume.
9. Restart nitrogen addition with slow vortex-free stirring. Stir for 20 minutes. Assure that the pH is 3-3.5.
10. Stop and remove the stirrer. Stop the nitrogen flow. Store the solution in the dark, under a nitrogen head protected from air.
11. Using aseptic and lint-free conditions for all the following and under a push of nitrogen, pass the solution through a 0.2 micron HT-Tuffryn polysulfone membrane filter (Gelman Sciences). Collect the filtrate in a suitable glass container and store in the dark under a nitrogen head.

12. Under nitrogen purge add the required amount of solution into suitable glass vials. Stopper the vials with teflon-coated stoppers and seal with aluminum seals.

Solutions prepared as above maintained full potency with virtually no increase in doxorubicinone degradation product (aglycone) when stored at 40°C for at least 1 month. This translates into a predicted shelf-life of >3 years at 4°C.

In a similar manner solutions having 0.5 mg/ml and 2 mg/ml sodium acetone bisulfite were prepared. These solutions also had a predicted shelf-life of >3 years at 4°C.

## Claims

1. A pharmaceutical formulation of a stable, sterile, ready-to-use aqueous solution in a sealed container suitable for parenteral administration containing 0.1 to 20 mg/ml of a physiologically acceptable salt of doxorubicin, a stabilizing amount of sodium acetone bisulfite of 0.1 to 10 mg/ml, water, and, if required, an amount of a physiologically acceptable acid or base necessary to adjust the solution pH to within the range of 2.5 to 6.0.

2. A formulation according to Claim 1 wherein the solution pH is in the range of 3.0 to 3.5.

3. A formulation according to Claim 1 wherein the solution pH is in the range of 4.5 to 6.0.

4. A formulation according to Claim 1, 2 or 3 wherein there is also present a tonicity adjustment agent.

5. A formulation according to Claim 1 containing per ml of solution, 2 mg doxorubicin hydrochloride, 1 mg sodium acetone bisulfite, and water q.s.

6. A formulation according to Claim 1 containing per ml of solution, 2 mg doxorubicin hydrochloride, 1 mg sodium acetone bisulfite, 0.2 mg of citric acid resulting in a pH of between 3.0 and 3.5, and water q.s.

7. A formulation according to Claim 1 containing per ml of solution, 2 mg doxorubicin hydrochloride, 1 mg sodium acetone bisulfite, an amount of hydrochloric acid sufficient to adjust the pH to between 3.0 and 3.5, and water q.s.

8. A formulation according to any of Claims 1, 2, 3, 4, 5, 6 or 7 wherein the air in the headspace of the container has been substantially replaced with an inert gas.

9. A method of preparing a pharmaceutical formulation of a stable, sterile, ready-to-use aqueous solution of a physiologically acceptable salt of doxorubicin suitable for parenteral administration, said method comprising the steps of (1) mixing 0.1 to 20 mg/ml of a physiologically acceptable salt of doxorubicin, a stabilizing amount of sodium acetone bisulfite of 0.1 to 10 mg/ml and water to form a solution, (2) adjusting, if necessary, the pH of said solution to within the range of 2.5 to 6.0 by adding a physiologically acceptable acid or base, (3) adding such solution to a container suitable for parenteral administration, and (4) sealing said container.

10. The method of Claim 9 wherein the solution pH is adjusted to within the range of 3.0 to 3.5.

11. The method of Claim 9 wherein the solution pH is adjusted to within the range of 4.5 to 6.0.

12. The method of Claim 9, 10 or 11 wherein in step (1) there is also added a tonicity adjustment agent.

13. The method of Claim 9 for producing a solution containing per ml of solution : 2 mg doxorubicin hydrochloride, 1 mg sodium acetone bisulfite, and water q.s.

14. The method of Claim 9 for producing a solution containing per ml of solution : 2 mg doxorubicin hydrochloride, 1 mg sodium acetone bisulfite, 0.2 mg of citric acid resulting in a pH of between 3.0 and 3.5, and water q.s.

15. The method of Claim 9 for producing a solution containing per ml of solution : 2 mg doxorubicin hydrochloride, 1 mg sodium acetone bisulfite, an amount of hydrochloric acid sufficient to adjust the pH to between 3.0 and 3.5, and water q.s.

16. The method according to any Claims 9-15 wherein, just prior to step (4), the air in the headspace of the container is substantially replaced by an inert gas.

## Patentansprüche

1. Pharmazeutische Formulierung aus einer stabilen, sterilen, gebrauchsfertigen, wäßrigen Lösung in einem geschlossenen, zur parenteralen Anwendung geeigneten Behälter, enthaltend 0,1 bis 20 mg/ml eines Physiologisch verträglichen Salzes von Doxorubicin, eine stabilisierende Menge Natriumacetonbisulfit von 0,1 bis 10 mg/ml, Wasser und, falls nötig, eine zur Einstellung des pH-Wertes der Lösung im Bereich von 2,5 bis 6,0 erforderliche Menge einer physiologisch verträglichen Säure oder Base.

2. Formulierung nach Anspruch 1, worin der pH-Wert der Lösung im Bereich von 3,0 bis 3,5 liegt.

3. Formulierung nach Anspruch 1, worin der pH-wert der Lösung im Bereich von 4,5 bis 6,0 liegt.

4. Formulierung nach Anspruch 1, 2 oder 3, worin zusätzlich ein Mittel zur Einstellung der Tonizität vor-

EP 0 372 888 B1

handen ist.

5. Formulierung nach Anspruch 1, die pro ml Lösung 2 mg Doxorubicinhydrochlorid, 1 mg Natriumacetonbisulfit und Wasser q.s. umfaßt.

6. Formulierung nach Anspruch 1, die pro ml Lösung 2 mg Doxorubicinhydrochlorid, 1 mg Natriumacetonbisulfit, 0,2 mg Zitronensäure, die zu einem pH von 3,0 bis 3,5 führt, und Wasser q.s. umfaßt.

7. Formulierung nach Anspruch 1, die pro ml Lösung 2 mg Doxorubicinhydrochlorid, 1 mg Natriumacetonbisulfit, eine zur Einstellung des pH-Wertes zwischen 3,0 und 3,5 erforderliche Menge Salzsäure und Wasser q.s. umfaßt.

8. Formulierung nach einem der Ansprüche 1, 2, 3, 4, 5, 6 oder 7, worin die Luft im Kopfteil des Behälters im wesentlichen durch ein Edelgas ersetzt worden ist.

9. Verfahren zur Herstellung einer pharmazeutischen Formulierung aus einer stabilen, sterilen, gebrauchsfertigen, wäßrigen Lösung eines physiologisch verträglichen, zur parenteralen Verabreichung geeigneten Doxorubicinsalzes, wobei man (1) 0,1 bis 20 mg/ml eines physiologisch akzeptabeln Doxorubicinsalzes, eine stabilisierende Menge Natriumactonbisulfit von 0,1 bis 10 mg/ml und Wasser Vermischt, wobei man eine Lösung erhält, (2) falls nötig, den pH der Lösung im Bereich von 2,5 bis 6,0 einstellt, indem man eine physiologisch verträgliche Säure oder Base zugibt, (3) diese Lösung in einen zur parenteralen Verabreichung geeigneten Behälter gibt, und (4) den Behälter verschließt.

10. Verfahren nach Anspruch 9, worin der pH der Lösung im Bereich von 3,0 bis 3,5 eingestellt wird.

11. Verfahren nach Anspruch 9, worin der PH der Lösung im Bereich von 4,5 bie 6,0 eingestellt wird.

12. Verfahren nach Anspruch 9, 10 oder 11, worin in Stufe (1) zusätzlich ein Mittel zur Einstellung der Tonizität zugegeben wird.

13. Verfahran nach Anspruch 9 zur Herstellung einer Lösung, die pro ml Lösung 2 mg Doxorubicinhydrochlorid, 1 mg Natriumacetonbisulfit und Wasser q.s. umfaßt.

14. Verfahren nach Anspruch 9 zur Herstellung einer Lösung, die pro ml Lösung 2 mg Doxorubicinhydrochlorid, 1 mg Natriumacetonbisulfit, 0,2 mg Zitronensäure, die einen pH-Wert von 3,0 bis 3,5 ergibt, und Wasser q.s. umfaßt.

15. Verfahren nach Anspruch 9 zur Herstellung einer Lösung, die pro ml Lösung 2 mg Doxorubicinhydrochlorid, 1 mg Natriumacetonbisulfit, eine zur Einstellung des pH von 3,0 bis 3,5 erforderliche Menge Salzsäure und Wasser q.s. umfaßt.

16. Verfahren nach einem der Ansprüche 9 - 15, worin die Luft im Kopfteil des Behälters unmittelbar vor Stufe (4) durch ein Edelgas ersetzt wird.


**Revendications**

1. Une formulation pharmaceutique d'une solution aqueuse prête à l'emploi stérile stable dans un récipient fermé hermétiquement convenable pour l'administration parentérale contenant 0,1 à 20 mg/ml d'un sel acceptable du point de vue physiologique de la doxorubicine, une quantité stabilisante d'acétone bisulfite de sodium de 0,1 à 10 mg/ml, de l'eau et, si cela est nécessaire, une quantité d'un acide ou d'une base acceptable du point de vue physiologique nécessaire pour ajuster le pH de la solution dans la gamme de 2,5 à 6,0.

2. Une formulation suivant la revendication 1, dans laquelle le pH de la solution est dans la gamme de 3,0 à 3,5.

3. Une formulation suivant la revendication 1, dans laquelle le pH de la solution est dans la gamme de 4,5 à 6,0.

4. Une formulation suivant l'une quelconque des revendications 1, 2 ou 3, dans laquelle il y a également un agent pour l'ajustement de la tonicité.

5. Une formulation suivant la revendication 1, contenant par ml de solution, 2 mg de chlorhydrate de doxorubicine, 1 mg d'acétone bisulfite de sodium et de l'eau en quantité suffisante.

6. Une formulation suivant la revendication 1, contenant par ml de solution, 2 mg de chlorhydrate de doxorubicine, 1 mg d'acétone bisulfite de sodium, 0,2 mg d'acide citrique fournissant un pH compris entre 3,0 et 3,5, et de l'eau en quantité suffisante.

7. Une formulation suivant la revendication 1, contenant par ml de solution, 2 mg de chlorhydrate de doxorubicine, 1 mg d'acétone bisulfite de sodium, une quantité d'acide chlorhydrique suffisante pour ajuster le pH à une valeur comprise entre 3,0 et 3,5, et de l'eau en quantité suffisante.

8. Une formulation suivant l'une quelconque des revendications 1 à 7, dans laquelle l'air de l'espace supérieur du récipient a été pratiquement remplacé par un gaz inerte.

9. Un procédé pour préparer une formulation pharmaceutique d'une solution aqueuse prête à l'emploi stérile stable d'un sel acceptable du point de vue physiologique de doxorubicine convenable pour l'administration

6

parentérale, ce procédé comprenant les étapes de: (1) mélange de 0,1 à 20 mg/ml d'un sel acceptable du point de vue physiologique de doxorubicine, d'une quantité stabilisante d'acétone bisulfite de sodium de 0,1 à 10 mg/ml, et d'eau pour former une solution, (2) ajustement, si cela est nécessaire, du pH de cette solution à une gamme de 2,5 à 6,0 par addition d'un acide ou d'une base acceptable du point de vue physiologique, (3) introduction de cette solution dans un récipient convenable pour l'administration parentérale, et (4) fermeture hermétique de ce récipient.

10. Le procédé suivant la revendication 9, dans lequel le pH de la solution est ajusté dans la gamme de 3,0 à 3,5.

11. Le procédé suivant la revendication 9, dans lequel le pH de la solution est ajusté dans la gamme de 4,5 à 6,0.

12. Le procédé suivant la revendication 9, 10 ou 11, dans lequel, dans l'étape (1), on ajoute également un agent pour l'ajustement de la tonicité.

13. Le procédé suivant la revendication 9, pour produire une solution contenant par ml de solution: 2 mg de chlorhydrate de doxorubicine, 1 mg d'acétone bisulfite de sodium et de l'eau en quantité suffisante.

14. Le procédé suivant la revendication 9, pour produire une solution contenant par ml de solution: 2 mg de chlorhydrate de doxorubicine, 1 mg d'acétone bisulfite de sodium, 0,2 mg d'acide citrique fournissant un pH compris entre 3,0 et 3,5 et de l'eau en quantité suffisante.

15. Le procédé suivant la revendication 9, pour produire une solution contenant par ml de solution: 2 mg de chlorhydrate de doxorubicine, 1 mg d'acétone bisulfite de sodium, une quantité d'acide chlorhydrique suffisante pour ajuster le pH à une valeur comprise entre 3,0 et 3,5 et de l'eau en quantité suffisante.

16. Le procédé suivant l'une quelconque des revendications 9 à 15, dans lequel, juste avant l'étape (4), l'air se trouvant dans l'espace supérieur du récipient est pratiquement remplacé par un gaz inerte.